# EUROPÄISCHE PATENTSCHRIFT

(11) **EP 0 351 406 B1**
(45) Veröffentlichungstag und Bekanntmachung des Hinweises auf die Patenterteilung: **22.12.1993**
(21) Anmeldenummer: 88902133.3
(22) Anmeldetag: 03.03.1988
(51) Int. Cl.: A61K 39/21

(54) **MITTEL ZUR VERHINDERUNG DER PROGREDIENZ DER ERKRANKUNG NACH HIV-VIRUSINFEKTIONEN**
AGENT PREVENTING THE DEVELOPMENT OF DISEASE AFTER HIV INFECTION
AGENT ARRETANT L'EVOLUTION DE MALADIES PROVOQUEES PAR DES INFECTIONS AVEC DES HIV

(30) Priorität: 06.03.1987 DE 3707260
(43) Veröffentlichungstag der Anmeldung: 24.01.1990
(73) Patentinhaber: DIAGEN Institut für molekularbiologische Diagnostik GmbH, D-40724 Hilden (DE)
(72) Erfinder: BRÜSTER, Herbert, Theodor, D-4040 Neuss (DE)
(74) Vertreter: Werner, Hans-Karsten, Dr.Dipl.-Chem.
(86) Internationale Anmeldenummer: EP8800162
(87) Internationale Veröffentlichungsnummer: WO8806454

(56) Entgegenhaltungen:
- Biological Abstracts, vol. 83, no.3, 1987, Biosciences Information Service (Philadelphia, Pennsylvania, US), P.A. Marx et al.: "Prevention of simian acquired immune deficiency syndrome with a formalin-inactivated type D retrovirus vaccine ", see abstract no. 24215
- Nature, vol. 327, 11 June 1987, J. Salk: "Prospectus for the control fo AIDS by immunizing seropositive individuals", see pages 473-476

## Beschreibung

Gegenstand der vorliegenden Erfindung ist ein Mittel zur Verhinderung der Progredienz der Erkrankung nach HIV-Virusinfektionen sowie ein verfahren zur Herstellung des Mittels.

AIDS ist eine tödlich verlaufende, durch HIV Viren hervorgerufene Erkrankung des Immunsystems und aufgrund der pandemisch verlaufenden Durchseuchung zu einer ernsthaften Bedrohung für die Menschheit geworden. Ein Krankheitsbild, das häufig auch als die eigentliche Ursache der Immunschwäche angesehen wird, äußert sich in dem Befall einer Lymphozytensubpopulation, den T4-Helferzellen, des Immunsystems mit den HIV-Viren. In den Stadien vor der letalen Phase der Erkrankung läßt sich die Abnahme der T4-Zellen beobachten. Dadurch bedingt, verschiebt sich auch das T4/T8-Verhältnis zu ungünstigen Werten (die T8-Zellen, die sogenannten Suppressorzellen, bilden eine weitere Subpopulation der T-Lymphozyten).

Die Bemühungen der Fachwelt, diese Erkrankung zu bekämpfen, hat zu verschiedensten Ansätzen, angefangen mit präventiven Maßnahmen bis hin zu Versuchen, Impfstoffe zu gewinnen, geführt. Dabei bediente man sich klassischer und moderner Methoden zur versuchsweisen Herstellung einer Vakzine.

Vakzinierungen bei HIV-Infektionen haben sich bislang als problematisch erwiesen. Es wurden Peptide, Proteinfragmente und größere Proteine, die als antigene Determinanten bekannt sind, versuchsweise zur Herstellung von Vakzinen eingesetzt.

Dabei wurden verschiedene Wege beschritten. So kann beispielsweise eine Vakzine durch Züchtung virulenter Stämme, attenuierter Virusstämme oder apathogener Stämme, die proteinsynthetische Antigengewinnung oder die Expression antigener Strukturen in rekombinanten pro- oder eukaryotischen Zellen entwickelt werden. Die Verfahren weisen den Nachteil auf, daß das antigene Produkt als Protein oder Peptid eine definierte Struktur bzw. Sequenz besitzt. Man erhält dann eine Vakzine, die nur auf speziellen, exprimierten Antigenen beruht, die also nicht zur Bildung von Antikörpern gegen alle möglichen Variationen der Antigenstruktur führt.

Das HIV-Virus zeichnet sich durch eine extreme Variabilität aus, die an bekannte Phänomene im Zusammenhang mit Malaria-Erregern und Trypanosomen erinnert. Bei den Malaria- und Trypanosoma-Infektionen konnten die Mechanismen recht gut aufgeklärt werden: Wann immer der infizierte Organismus Antikörper synthetisiert hat, die gegen die Oberflächenantigene des Krankheitserregers gerichtet sind und neutralisierend wirken, greift der Einzeller auf neue, genomisch determinierte Antigene zurück. Somit werden neue Epitope präsentiert. Es dauert dann eine gewisse Zeit, bis das Immunsystem auf diese Veränderungen in der Antigenstruktur der Erreger erneut reagieren kann. Danach beginnt der Krankheitszyklus erneut.

Bei HIV-Virusinfektionen wird hingegen ein anderer Mechanismus benutzt, allerdings mit ähnlicher Konsequenz: Aufgrund ständiger Ablesefehler der reversen Transkriptase dieses Retrovirus gleicht kaum eine Viruskopie der anderen.

Obwohl offensichtlich von einem gemeinsamen Vorläufer abstammend, werden innerhalb weniger Generationen Virusvarianten produziert, die um mehrere Prozent (bis zu 30% und mehr) auf der genetischen Ebene voneinander abweichen. Divergente Virusvarianten werden selbst zu einer definierten Zeit im selben Patienten angetroffen. So sind aus einem Patienten zu einer bestimmten Zeit vier unterschiedliche Virusvarianten isoliert worden. Die tatsächliche Anzahl ist sicherlich sehr viel höher. Das erklärt die bisherige Erfolglosigkeit der Vakzinierungsexperimente mit einer einzigen, auf molekularer Ebene definierten antigenen Struktur, die zu einer entsprechend definierten Vakzine führt.

Die Variabilität des HIV-Virus führt zu solcher Divergenz, daß ohne das inzwischen angehäufte Wissen über HIV und seine Entwicklung HIV-Varianten als separate Viren benannt werden könnten. Durch diese Heterogenität bedingt, werden nicht nur immunologisch differierende Antigenvarianten diskutiert, sondern auch veränderte Tropismen der Viren, die zum Beispiel mit der neurologischen Krankheitssymptomatik korrelieren könnten.

Eine Immunisierung ist daher dann sinnlos, wenn aus dem großen Spektrum der Varianten einige wenige über mutierte Antigene verfügen, die eine biologische Neutralisation durch bereits vorhandene Antikörper nicht zulassen, zumal das Immunsystem in der Progredienz der Erkrankung insuffizient wird. Es ist inzwischen bekannt, daß schon niedrigste Virustiter von HIV bei einmaliger Virusexposition zu einer Infektion führen können. Wenn aber auch nur wenige Viren der immunologischen Eliminierung entgehen, kann auch die Infektion persistieren. Bei der hohen Mutationsfrequenz der HIV-Viren ist weiterhin mit einem schnellen Adaptationsprozeß der Viren an den immunologischen Selektionsdruck zu rechnen, da gerade die Varianten angereichert werden, die der Eliminierung durch das Immunsystem entgehen.

In der US-PS 3,735,004 wird eine Methode zur Inaktivierung von Hepatitis-B-Viren durch kochendes, die Viren enthaltendes Serum bei 98 bis 100°C beschrieben. Durch diese Verfahrensweise verlieren die Viren ihre infektiösen Eigenschaften. Die antigenen Eigenschaften bleiben jedoch erhalten. Die DE-OS 28 45 399 betrifft ein Verfahren und eine Vorrichtung zur Auftrennung von Blut in Fraktionen, zum Beispiel Thrombozyten. Im wesentlichen besteht die beschriebene Vorrichtung aus einer Zwei-Kammer-Schleudereinrichtung, die zur Auftrennung des Blutes eingesetzt wird.

In A. Mayr u.a., "Virologische Arbeitsmethoden", Gustav Fischer Verlag, Stuttgart 1974, Band 1, Seite 30 werden allgemeine Methoden zum Aufschluß von mit Viren befallenen Zellen beschrieben. Unter anderem werden Methoden wie Gefriertauen und Ultraschallbehandlungen erwähnt. In H. Zeichhardt u.a., Bundesgesundheitsblatt 30, Nr. 5, 1987, Seiten 172-177 wird die Inaktivierung der HIV-Viren durch Wärmebehandlung bei 60°C beschrieben.

Die Aufgabe der vorliegenden Erfindung besteht darin, ein Mittel zur Verfügung zu stellen, das in der Lage ist, als Vakzine zu dienen, die alle aktuellen, zu einem bestimmten Zeitpunkt in dem zu behandelnden Patienten vorliegenden HIV-Virusantigene umfaßt. Durch den optimalen Abgleich zwischen den immunogenen HIV-Antigen-Varianten, den daraufhin gebildeten Antikörpern und den zu attackierenden Viren und Virusantigen exprimierenden Zellen wird eine effektive und spezifische Vakzine erhalten.

Weiterhin soll die vorliegende Erfindung zu einer Vakzine führen, die auch bei sogenannten "non-respondern" effektiv ist. Als "non-responder" werden Patienten bezeichnet, die von einem pathogenen HIV-Virus infiziert sind, jedoch keine Antkörper bilden und auch nicht zu einer zellulären Immunantwort befähigt sind, obwohl sie ansonsten über ein intaktes Immunsystem verfügen. Die Ursache hierfür könnte in dem sogenannten spezifischen Haplotyp des Patienten liegen, nämlich in der Unfähigkeit, bestimmte Virusvarianten in Assoziation mit den Histokompatibilitätsantigenen dem Immunsystem so zu präsentieren, daß eine Immunantwort resultiert. Eine ähnliche Situation ist im Verlauf einer Erkrankung denkbar, wenn eine Virusvariante entsteht, der das Immunsystem als "non-responder", wie oben beschrieben, gegenübersteht.

Durch die indirekte physikalische Behandlung könnten auch solche Antigene in Immunogene umgewandelt werden, das heißt, durch Strukturänderungen werden solche Antigene auch immunogen. Darüber hinaus ist es durchaus denkbar, daß die positive Wirkung des erfindungsgemäß hergestellten Mittels nicht oder nicht ausschließlich auf der Freisetzung und/oder den Modifikationen des viralen antigenen Materials beruht. Es könnten allein oder in Kombination ein oder mehrere zelluläre Bestandteile oder Zellinhaltsstoffe freigesetzt werden, die zu den gewünschten Effekten positiv beitragen. Es ist sogar denkbar, daß die Wirkung ebenfalls erzielt werden kann durch Verwendung eines Mittels, das aus Spenderblut von gesunden Personen nach dem erfindinngsgemäßen Verfahren gewonnen wurde. Der Effekt könnte dabei auf immunmodulatorischer Wirkung, selbst einer immunsuppressorischen Wirkung, beruhen.

Es ist somit ein weiteres Ziel der vorliegenden Erfindung, ein Verfahren zur Herstellung des oben beschriebenen Mittels zur Verfügung zu stellen.

Erfindungsgemäß wird die Aufgabe gelöst durch ein Mittel zur Verhinderung der Progredienz der Erkrankung nach HIV-Virusinfektionen, erhältlich durch Behandlung von aus HIV-infizierten Patienten entnommenem Blut, das in Lymphozyten enthaltende Fraktionen getrennt wird, dadurch gekennzeichnet, daß diese Fraktionen mit indirekten physikalischen Methoden behandelt werden in Form von sequenzieller Frier-/ Tau-/Thermobehandlung und/oder Ultraschall-/Thermobehandlung und/oder Kombination von Gefrier-/Tau- und Ultraschall-/Thermobehandlung, wobei die Thermobehandlung bei mindestens 45°C durchgeführt wird.

Das erfindungsgemäße Verfahren wird eingeleitet durch Entnahme des Blutes des Patienten. Bevorzugt wird die Entnahme von 300 bis 500 ml Blut. Dieses kann danach in Fraktionen getrennt werden, beispielsweise mittels eines Zellseparators. Die Erythrozyten und Serumfraktionen werden wieder vereinigt und können gemäß einer Ausführungsform des erfindungsgemäßen Verfahrens direkt wieder in den Blutkreislauf des Patienten zurückgeführt werden. Die isolierten Fraktionen enthalten HIV-infizierte Zellen, vorwiegend Lymphozyten.

Die so gewonnenen Fraktionen aus dem Patientenblut werden einem sequentiellen Frier-/Auftauprozeß unterzogen, um die Zellen aufzubrechen. Dabei wird ein Temperaturintervall von Raumtemperatur bis -190°C durchlaufen. Vorzugsweise kühlt man in einem Gefrierschrank bis -80°C und läßt dann auf 4°C auftauen. Dies kann mehrmals wiederholt werden.

Das Aufbrechen der Zellen kann auch durch Ultraschallbehandlung oder andere mechanische Mittel erfolgen.

Danach werden die daraus gewonnenen Fraktionen einer Wärmebehandlung bei mindestens 45°C, vorzugsweise 56°C für eine Dauer von 20 Minuten bis zu mehreren Stunden, vorzugsweise 30 Minuten bis 10 Stunden unterzogen.

Es können gewünschtenfalls nach der Temperaturbehandlung noch weitere Trennoperationen, zum Beispiel Zentrifugationen bei niedriger Umdrehungszahl, vorzugsweise 3000 x g für 30 Minuten, und/oder Filtration der erhaltenen Flüssigkeit erfolgen. Das erfindungsgemäß erhaltene Mittel ist eine Infusionslösung, die dem Patienten wieder verabreicht werden kann. Gewünschtenfalls werden zuvor langkettige Nukleinsäuren mit potentiell infektiösem oder onkogenem Potential weitgehend abgetrennt oder entfernt.

Das Verfahren ist in einer bevorzugten Ausführungsform so ausgestaltet, daß sterile Materialien für den Einmalgebrauch eingesetzt werden können. Die Verfahrensschritte können darüber hinaus so optimiert werden, daß sie automatisierbar sind.

Das erfindungsgemäße Verfahren gewährleistet nicht nur eine optimale Präsentation der viralen Antigene, sondern bewirkt auch, daß die Vakzinelösung nach den beschriebenen Manipulationen mit den physikalischen Methoden nicht mehr infektiös ist. Selbst wenn infizierte Lymphozyten dem Patienten mit der Infusionslösung wieder verabreicht werden sollten, sind diese nicht mehr replikationsfähig und stehen somit als persistierender Virusspeicher und/oder Virusproduzent nicht mehr zur Verfügung. Die antigenen Eigenschaften bereits synthetisierter Antigene bleiben jedoch erhalten.

Ein entscheidender Vorteil der Anwendung des erfindungsgemäßen Mittels ist die Tatsache, daß bereits die erste Immunisierung eine Boosterung darstellt. Der Organismus wird, ohne daß kostbare Zeit verstreicht, in der neue Varianten produziert werden, mit dem Gesamtkollektiv seiner eigenen Varianten, jedoch mit offensichtlich verstärktem antigenen Potential konfrontiert. Als Ergebnis wird das Immunsystem gezielt sensibilisiert, nämlich exakt gegen die noch vorhandenen zirkulierenden infektiösen Varianten gleichen Antigenmusters. Es kann stark bezweifelt werden, ob eine Vakzinierung mit dem gleichen Präparat, jedoch zeitversetzt zu einem erheblich späteren Zeitpunkt erfolgreich sein kann, wie es Von herkömmlichen Beispielen von Autoimmunisierungen bekannt ist.

Noch ein weiterer Vorteil des erfindungsgemäßen Mittels ist darin zu sehen, daß quasi maßgeschneiderte Vakzine für den jeweiligen HIV-infizierten Patienten erhalten werden können. Es ist selbstverständlich auch möglich, eine auf die beschriebene Weise erhaltene Fraktion einem anderen mit HIV-Viren infizierten Patienten zu infundieren.

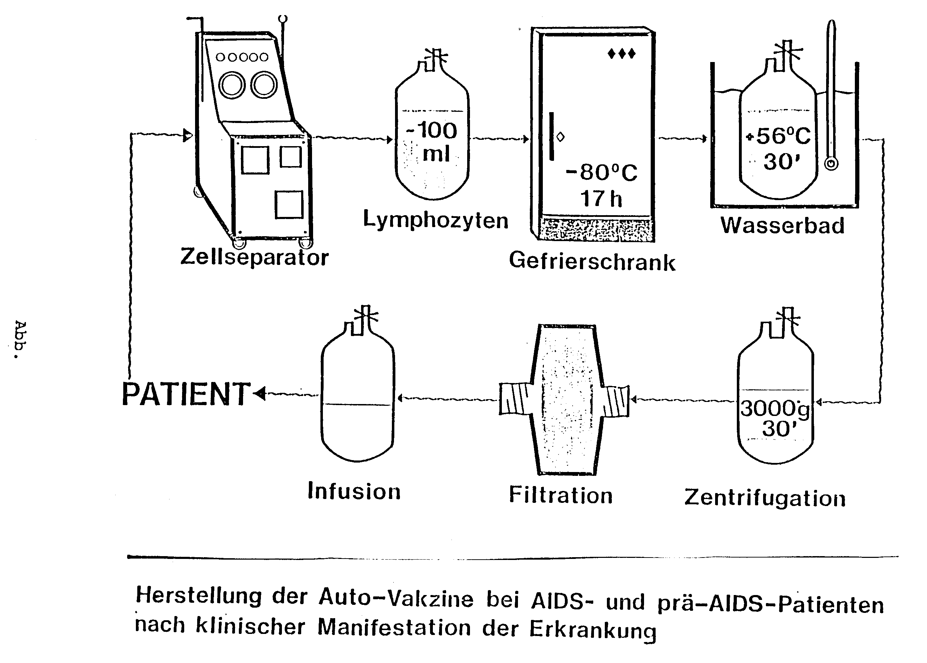

Eine einfache Ausführungsform des erfindinngsgemäßen Verfahrens zur Herstellung des erfindinngsgemäßen Mittels wird in der Abbildung veranschaulicht. Dort sind die relevanten Verfahrensschritte aufgeführt. Es ist auch möglich, direkt nach der Wärmebehandlung zu infundieren und auf die vorherige Zellseparation zu verzichten.

Das folgende Beispiel dient der exemplarischen Erläuterung des erfindungsgemäßen Verfahrens.

### Beispiel

Sobald ein Patient einen HIV-positiven Immunstatus aufweist, kann mit der Herstellung und Applikation der Autoimmunvakzine begonnen werden. Die Behandlung kann sogar noch beim Eintritt in das Prä-AIDS-Stadium, dessen meßbarer Parameter im T4/T8-Lymphozyten-Verhältnis einen Wert von kleiner als 0,5 darstellt, erfolgen.

Dem Patienten werden ca. 350 bis 400 ml Blut entnommen, mit handelsüblichen Antikoaginlantien (ACDB; siehe United States Pharmacopeia (USP 21)) versetzt und dann direkt in einem handelsüblichen Zellseparator prozessiert. Dabei wird dem Patienten das Erythrozytenkonzentrat sofort wieder infundiert, wie auch die Plasmafraktionen. Lediglich die Fraktion mit den angereichterten Lymphozyten ("buffy coat") im Serum (ca. 100 ml) wird im 600-ml-Blutbeutel gesammelt und steril verschweißt. Diese Fraktion ist das Ausgangsmaterial zur Herstellung der sogenannten Autovakzine.

Die sogenannte "Kältesprengung" der Zellen wird wie folgt durchgeführt: Der Blutbeutel wird flach in einen -80°C Gefrierschrank gelegt, um einen raschen Gefrierprozeß zu gewährleisten, denn der gefüllte Blutbeutel ist nur 0,3 bis 0,5 cm dick. Schockfrierungen in flüssigem Stickstoff und in Ethanol/Trockeneis können ebenfalls durchgeführt werden.

Nach 17 Stunden bei -80°C wird der Beutel in einem 0 bis 4°C warmen Wasserbad getaut und der Gefrier-/Tau-Zyklus zweimal wiederholt und/oder die Zellsprengung vervollständigt durch eine Ultraschallbehandlung (15 Minuten bei 0 bis 4°C, Frequenz: 45 kHz, 120 bis 140 Watt im Wasserbad).

Eine mikroskopische Analyse des Inhalts eines Testbeutels ergibt bereits nach diesen Schritten eine nahezu vollständige Lyse der zellulären Bestandteile (Leukozyten, Thrombozyten etc.).

Zur Abtötung virulenter HIV-Viren oder deren Aggregaten wird der Beutel für eine Zeit von 30 Minuten bis zu 10 Stunden bei 56°C ± 1°C im Wasserbad temperiert. Danach kann kein infektiöses Virus mehr nachgewiesen werden. Im Falle, daß anfangs dem Blut Stabilisatoren wie Saccharose, Glycerin etc. beigefügt werden, lassen sich auch deutlich höhere Temperaturen realisieren, ohne daß Faktoren und Enzyme nennenswert an Funktionsfähigkeit einbüßen.

Durch Zentrifugation bei 3000 x g für 30 Minuten werden die Zellfragmente abzentrifugiert. Der überstand wird durch einen sterilen Filter (5 bis 160 µm) in einen Zwillingsbeutel überführt. Von anfänglich ca. 100 ml bleiben 70 bis 80 ml Infusionslösung übrig, die nun dem Patienten intravenös infundiert werden. Die Infusionslösungen sind unmittelbar nach Herstellung zur Applikation geeignet oder können tiefgefroren gelagert und nach Ablauf von bis zu zwei Monaten und mehr verarbeitet werden.

Zu den klinischen Resultaten läßt sich folgendes ausführen:
14 Patienten wurden behandelt. Die Behandlungsdauer beläuft sich bei einigen Patienten auf bis zu zwei Jahre. Die sogenannte Prä-AIDS-Symptomatik hat sich bei allen Patienten klinisch deutlich verbessert. Die Behandlung wurde im Abstand von 6 Monaten bis zu einem Jahr wiederholt, gefolgt von jeweiligen Booster-Injektionen.

Sekundärinfektionen der Atemwege und Virusinfekte waren signifikant rückläufig, die Patienten erlangten ihren Lebensmut zurück und nahmen auch teilweise ihre unterbrochenen Berufstätigkeiten wieder auf. Die zellulären pathologischen Ungleichgewichtszustände beginnen sich zu normalisieren; insbesondere beginnt sich das T4/T8-Verhältnis zu normalisieren.

## Patentansprüche

1. Mittel zur Verhinderung der Progredienz der Erkrankung nach HIV-Virusinfektionen, erhältlich durch Behandlung von aus HIV-infizierten Patienten entnommenem Blut, das in Lymphozyten enthaltende Fraktionen getrennt wird, dadurch gekennzeichnet, daß diese Fraktionen mit indirekten physikalischen Methoden behandelt werden in Form von sequenzieller Frier-/Tau-/Thermobehandlung und/oder Ultraschall-/Thermobehandlung und/oder Kombination von Gefrier-/Tau- und Ultraschall-/Thermobehandlung, wobei die Thermobehandlung bei mindestens 45°C durchgeführt wird.

2. Mittel nach Anspruch 1, dadurch gekennzeichnet, daß anschließend an die indirekte physikalische Behandlung eine Zentrifugation und/oder Filtration durchgeführt wird.

3. Mittel nach Anspruch 1 oder 2, dadurch gekennzeichnet, daß die Fraktionen aus dem HIV-infizierten Patientenblut T₄/T₈ Lymphozyten enthält.

4. Mittel nach einem der Ansprüche 1 bis 3, dadurch gekennzeichnet, daß sich nach Zugabe von HIV-sensiblen Zellen keine HIV-Viren züchten lassen.

5. Mittel nach einem der Ansprüche 1 bis 4, dadurch gekennzeichnet, daß die langkettigen Nukleinsäuren mit potentiell infektiösem oder onkogenem Potential größtenteils entfernt worden sind.

6. Mittel nach einem der Ansprüche 1 bis 5, dadurch gekennzeichnet, daß immunkompetente "non-responder" zur Immunantwort auf eine HIV-Infektion angeregt werden.

7. Verfahren zur Herstellung eines Mittels zur Verhinderung der Progredienz der Erkrankung nach HIV-Virus-Infektionen, dadurch gekennzeichnet, daß das einem HIV-infizierten Patienten entnommene Blut fraktioniert wird, die Lymphozyten enthaltenden Fraktionen mit indirekten physikalischen Methoden behandelt werden in Form mindestens eines Frier-/Tauprozesses und/oder mindestens einer Ultraschallbehandlung und einer Thermobehandlung bei mindestens 45°C.

8. Verfahren nach Anspruch 7, dadurch gekennzeichnet, daß die Lymphozytenfraktion T₄/T₈ Lymphozyten enthält.

9. Verfahren nach Anspruch 7 oder 8, dadurch gekennzeichnet, daß die Thermobehandlung bei einer Temperatur von mindestens 56°C durchgeführt wird.

10. Verfahren nach einem der Ansprüche 7 bis 9, dadurch gekennzeichnet, daß die Fraktionen mittels eines Zellseparators und/oder einer Zentrifugation gewonnen werden.

11. Verfahren nach einem der Ansprüche 7 bis 10, dadurch gekennzeichnet, daß als nachgeordneter Verfahrensschritt eine Zentrifugation und/oder Filtration durchgeführt wird.

12. Verfahren nach einem der Ansprüche 7 bis 11, dadurch gekennzeichnet, daß vor der indirekten physikalischen Behandlung ein gerinnungshemmender Stoff zugegeben wird.

13. Verfahren nach einem der Ansprüche 7 bis 12, dadurch gekennzeichnet, daß die Gewinnung des Mittels nach Anspruch 1 in einem geschlossenen System steriler Materialien für den Einmalgebrauch erfolgt.

14. Verwendung des Mittels nach Anspruch 1 zur Herstellung eines Arzneimittels zur Verhinderung der Progredienz der Erkrankung nach HIV-Virusinfektionen.

## Claims

1. An agent for preventing the progression of the disease after HIV virus infections, obtainable by the treatment of blood taken from a HIV-infected patient and separated into fractions containing lymphocytes, characterized in that said fractions are treated with indirect physical methods in the form of a sequential freeze/thaw/thermal treatment and/or ultrasonic/thermal treatment and/or a combination of freeze/thaw and ultrasonic/thermal treatment, the thermal treatment being carried out at at least 45 °C.

2. The agent according to claim 1, characterized in that a centrifugation and/or filtration is carried out subsequently to the indirect physical treatment.

3. The agent according to claims 1 or 2, characterized in that said fractions from the blood of the HIV-infected patient contain T4/T8 lymphocytes.

4. The agent according to anyone of claims 1 to 3, characterized in that no HIV viruses can be cultivated upon addition HIV-sensitive cells.

5. The agent according to anyone of claims 1 to 4, characterized in that the long-chain nucleic acid having a potentially infectious or oncogenetic potential have been removed to the largest part thereof.

6. The agent according to anyone of claims 1 to 5, characterized in that immune-competent "non-responders" are stimulated for an immune response to a HIV infection.

7. A process for preparing an agent for preventing the progression of the disease after HIV virus infections, characterized in that the blood taken from a HIV-infected patient is fractionated, the fractions containing lymphocytes are treated with indirect physical methods in the form of at least one freeze/thaw treatment and/or at least one ultrasonic treatment and a thermal treatment at at least 45 °C.

8. The process according to claim 7, characterized in that said lymphocyte fractions contain T4/T8 lymphocytes.

9. The process according to claims 7 or 8, characterized in that the thermal treatment is carried out at a temperature of at least 56 °C.

10. The process according to anyone of claims 7 to 9, characterized in that the fractions are recovered by means of a cell separator and/or by a centrifugation.

11. The process according to anyone of claims 7 to 10, characterized in that a centrifugation and/or filtration is/are carried out as subsequent process step(s).

12. The process according to anyone of claims 7 to 11, characterized in that an anticoagulant is added prior to the indirect physical treatment.

13. The process according to anyone of claims 7 to 11, characterized in that the preparation of the agent according to claim 1 is carried out in a closed system of disposable sterile materials.

14. Use of the agent according to claim 1 for the preparation of a medicament for preventing the progression of the disease after HIV virus infections.

## Revendications

1. Substance destinée à enrayer la progression des atteintes consécutives à des infections par virus HIV, cette substance pouvant être obtenue par traitement de sang prélevé sur les patients contaminés par virus HIV, qui est séparé en fractions contenant des lymphocytes,
caractérisé en ce que ces fractions sont traitées par des méthodes physiques indirectes consistant en un traitement thermique séquentiel de chauffage-congélationdécongélation, et/ou un traitement de chauffage et d'ultrasons et/ou une combinaison de traitement de chauffage-congélation-décongélation et de chauffage et d'ultrasons, le traitement de chauffage étant opéré à au moins 45°C.

2. Substance selon la revendication 1, caractérisée en ce qu'une centrifugation et/ou une filtration est opérée en combinaison avec le traitement par méthode physique indirecte.

3. Substance selon la revendication 1 ou 2, caractérisée en ce que la fraction de sang du patient contaminé par le virus HIV contiennent les lymphocytes T₄/T₈.

4. Substance selon l'une des revendications 1 à 3, caractérisée en ce qu'aucun virus HIV ne peut se développer après administration de cellules sensibles aux virus HIV.

5. Substance selon l'une des revendications 1 à 4, caractérisée en ce que les acides nucléiques à longue chaîne à potentiel éventuellement infectieux ou oncogène sont éliminés en majeure partie.

6. Substance selon l'une des revendications 1 à 5, caractérisee en ce que des substances de non réponse, à compétence immunitaire, sont excitées par une réponse immunitaire à une infection HIV.

7. Procédé d'obtention d'une substance pour inhiber la progression des atteintes consécutives à des infections par virus HIV, caractérisé en ce que le sang prélevé sur un patient contaminé par virus HIV est fractionné, les fractions contenant des lymphocytes sont traitées par des méthodes physiques indirectes consistant en au moins un traitement de congélation-décongélation, et/ou un traitement par ultrasons, et un traitement thermique à au moins 45°C.

8. Procédé selon la revendication 7, caractérisé en ce que la fraction lymphocytaire contient des lymphocytes T₄/T₈.

9. Procédé selon la revendication 7 ou 8, caractérisé en ce que le traitement thermique est exécuté à une température d'au moins 56°C.

10. Procédé selon l'une des revendications 7 à 9, caractérisé en ce que les fractions sont obtenues au moyen d'une séparation de cellules et/ou par centrifugation.

11. Procédé selon l'une des revendications 7 à 10, caractérisé en ce qu'une étape ultérieure du procédé consiste en une centrifugation ou une filtration.

12. Procédé selon l'une des revendications 7 à 11, caractérisé en ce qu'une substance retardant la coagulation est ajoutée avant le traitement par méthode physique indirecte.

13. Procédé selon l'une des revendications 7 à 12, caractérisé en ce que l'obtention de la substance selon la revendication 1 est réalisée dans un système clos de matériel stérile à usage unique.

14. Utilisation de la substance selon la revendication 1 à l'obtention d'un médicament pour empêcher la progression des atteintes consécutives à une infection par virus HIV.
